# EUROPEAN PATENT APPLICATION

(11) **EP 2 316 818 A1**
(43) Date of publication of application: **04.05.2011**
(21) Application number: 09174888.9
(22) Date of filing: 03.11.2009
(51) Int. Cl.: C07C 227/18, C07C 229/56

(54) **Method for the preparation of 2-amino-6-ethylbenzoic acid**

(71) Applicant: Active Biotech AB, 220 07 Lund (SE)
(72) Inventor: Jansson, Karl, 247 55 Dalby (SE)
(74) Representative: Stenbäck, Maria Elisabeth

(57) **Abstract**

The present invention relates to a novel method for the production of 2-amino-6-ethylbenzoic acid wherein 7-amino-3-methylphtalide or 7-amino-3-hydroxy-3-methylphtalide is reduced to 2-amino-6-ethylbenzoic acid in water or in a mixture of a water miscible solvent and water at a pH of 7 or higher using a hydrogenation catalyst selected from Raney nickel and palladium based catalysts and using a hydrogen source selected from the group consisting of hydrogen gas, formic acid derivatives and cyclohexen. The invention also relates to 2-amino-6-ethylbenzoic acid produced with this method, and to use of such 2-amino-6-ethylbenzoic acid in the production of paquini-mod.

## Description

### Field of the invention

The present invention relates to a method for the production of 2-amino-6-ethylbenzoic acid.

### Background to the Invention

Paquinimod (N,5-diethyl-1,2-dihydro-4-hydroxy-1-methyl-2-oxo-N-phenyl-3-quinolinecarboxamide) belongs to a rather new class of immunomodulating drugs that are evaluated in clinical trials. Paquinimod is currently in clinical phase 2 testing against systemic lupus erythematosus (SLE). 2-Amino-6-ethylbenzoic acid (Compound **3** in Scheme 1 below) is the starting material in the synthesis of paquinimod, as described by Jönsson et al. 2-amino-6-ethylbenzoic acid **3** is also an important starting material for other pharmacologically active substances e.g. carbamoyl benzoates, as disclosed in US 4,873,232. It has been a problem that 2-amino-6-ethylbenzoic acid **3** is difficult to prepare and hence expensive. Despite the seemingly simple structure of 2-amino-6-ethylbenzoic acid **3** only low-yielding methods are described in the literature, including synthesis via 4-ethylisatin (US 4,873,232), or nitration of 2-ethylaniline (Hansch; Newman et al), as illustrated in Scheme 1 below), both methods which suffers from formation of unwanted regioisomers and low-yielding steps. The route via 4-ethylisatin comprises a cyclization reaction in concentrated sulphuric acid which has shown difficult to scale up. The route from 2-ethylaniline involves a hydrolysis in low yield of an ortho di-substituted benzonitrile.

### Scheme 1: Prior art Routes to 2-Amino-6-Ethylbenzoic Acid 3

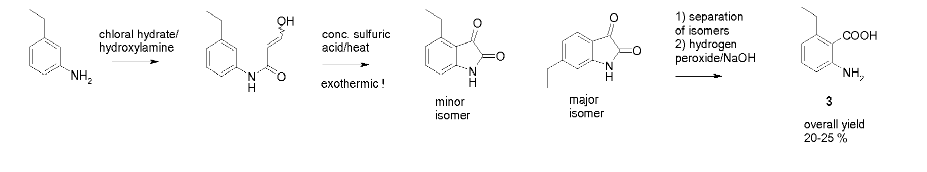

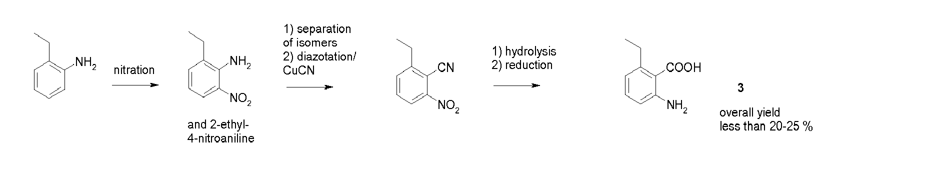

A new improved method for preparing 2-amino-6-ethylbenzoic acid **3** has therefore been sought after. Such a new method should be less expensive than prior art methods, and also more reliable. The poor reliability of prior art methods has resulted in delays in production of paquinimod. Furthermore, the malfunctioning prior art methods have resulted in high costs for raw materials.

### Summary of the Invention

One object of the present invention is a method for the production of 2-amino-6-ethylbenzoic acid wherein 7-amino-3-methylphtalide or 7-amino-3-hydroxy-3-methylphtalide is reduced to 2-amino-6-ethylbenzoic acid in water or in a mixture of a water miscible solvent and water at a pH adjusted to 7 or above using a hydrogenation catalyst selected from Raney nickel and palladium based catalysts and using a hydrogen source selected from the group consisting of hydrogen gas, formic acid derivatives and cyclohexen. When 7-amino-3-hydroxy-3-methylphtalide is used as the starting material, it is reduced to 2-amino-6-ethylbenzoic acid via *in situ* formation of 7-amino-3-methylphtalide.

Another object of the present invention is 2-amino-6-ethylbenzoic acid produced with the above mentioned method.

A further object is of the present invention is use of such 2-amino-6-ethylbenzoic acid in the production of N,5-diethyl-1,2-dihydro-4-hydroxy-1-methyl-2-oxo-n-phenyl-3-quinolinecarboxamide (paquinimod).

### Detailed Description of the Invention

In search for a starting material more useful for the preparation of 2-amino-6-ethylbenzoic acid **3,** a paper was found describing the synthesis of 2-acetyl-6-nitrobenzoic acid **5** in high yield from readily accessible 3-nitrophthalic acid (Luthy et al). This synthesis includes a highly regioselective condensation of diethylmalonate and 3-nitrophthalic anhydride, followed by hydrolysis and decarboxylation in aqueous hydrochloric acid to give 2-acetyl-6-nitrobenzoic acid **5** in 79 % overall yield. Although the paper did not explicitly describe the reduction of 2-acetyl-6-nitrobenzoic acid **5** into 2-amino-6-ethylbenzoic acid **3,** methods for the reduction of 2-acetyl-6-nitrobenzoic acid **5** into the closely related 7-amino-3-hydroxy-3-methylphtalide **7** and 7-amino-3-methylphtalide **6** were presented. Encouraged by this the inventor set out to find conditions for the reduction of 2-acetyl-6-nitrobenzoic acid **5** into 2-amino-6-ethylbenzoic acid **3.** However, it turned out that despite numerous trials with various catalysts and solvent, the reduction of 2-acetyl-6-nitrobenzoic acid **5,** or 7-amino-3-hydroxy-3-methylphtalide **7** or 7-amino-3-methylphtalide **6,** into 2-amino-6-ethylbenzoic acid **3,** was in most cases unsuccessful.

The study showed that the reduction follows the order **5-7-6-3** and that step **6** to **3** is a very slow process under neutral or acidic condition. Unexpectedly it was discovered during the study that reduction of **6** into **3** was very much accelerated at a pH of at least 7. This is further illustrated in Example 1 below.

Raney nickel was the first catalyst to be studied for the catalytic hydrogenation of 2-acetyl-6-nitrobenzoic acid **5** into 2-amino-6-ethylbenzoic acid **3.** This catalyst can be used in neutral or alkaline media but is unsuitable under acidic conditions where the catalyst dissolves. Reduction of 2-acetyl-6-nitrobenzoic acid **5** using RaNi in n-propanol gave a mixture of 7-amino-3-methylphtalide **6** and 7-amino-3-hydroxy-3-methylphtalide **7** and only small amounts of 2-amino-6-ethylbenzoic acid **3** (see Table 1 in experimental section). Under similar conditions but using a longer reaction time, 40 hours, the lactone **6** was formed as the major product (Luthy et al). When RaNi was used for the reduction of 2-acetyl-6-nitrobenzoic acid **5** in water at pH=12 the solution become very dark-colored and the reaction was inhibited due to catalyst inactivation. The resulting mixture was analyzed and consisted of starting material 2-acetyl-6-nitrobenzoic acid **5** and 7-amino-3-hydroxy-3-methylphtalide **7** and some unidentified byproducts. When Pd/C was used in water at pH 12 all 2-acetyl-6-nitrobenzoic acid **5** was transformed and gave 7-amino-3-hydroxy-3-methylphtalide **7** as the main product with no discoloration of the mixture, a result similar to that described before (Luthy et al) (see picture in Scheme 2. RaNi resulted in a dark-colored mixture whereas Pd/C gave no coloration). Use of Pd/C for the reduction of 2-acetyl-6-nitrobenzoic acid **5** in n-propanol gave 7-amino-3-methylphtalide **6** as the main product with only traces of 2-amino-6-ethylbenzoic acid **3.** Reduction of 7-amino-3-hydroxy-3-methylphtalide **7** with RaNi at pH=12 was successful and gave almost a complete conversion to 2-amino-6-ethylbenzoic acid **3** with no discoloration or byproduct formation. Pd/C or Pt was under the same conditions less effective leaving most of 7-amino-3-hydroxy-3-methylphtalide **7** unaffected. However, both Pd/C and RaNi were very good catalysts in water at pH=12 for the reduction of 7-amino-3-methylphtalide **6** into 2-amino-6-ethylbenzoic acid **3.** Platinum is not useful for this latter transformation since 7-amino-3-methylphtalide **6** was almost unaffected. Having learnt that lactone **6** was reduced into 2-amino-6-ethylbenzoic acid **3** only at a pH of 7 or above it was easy to predict that the known compound 3-methyl-7-nitrophtalide **9** would be amenable to the same reduction under similar conditions. In fact, 3-methyl-7-nitrophtalide **9,** prepared in high yield from 2-acetyl-6-nitrobenzoic acid **5** using sodium borohydride (Luthy et al), was successfully reduced to 2-amino-6-ethylbenzoic acid **3** with Pd/C in water at pH=12 presumable via lactone **6**.

In summary, during the work leading to the present invention it was thus found that the lactone **6**, shown below, which is very hard to reduce under traditional conditions, could be reduced to the desired 2-amino-6-ethylbenzoic acid **3** at a pH of 7 or above in water by using Raney nickel or palladium.

Accordingly, the present invention provides a method for the production of 2-amino-6-ethylbenzoic acid by reduction of 7-amino-3-methylphtalide **6** to 2-amino-6-ethylbenzoic acid **3** in water or in a mixture of a water miscible solvent and water at a pH of 7 or above using a palladium or a Raney nickel hydrogenation catalyst and a hydrogen source selected from the group consisting of hydrogen gas, formic acid derivatives and cyclohexen.

### Scheme 2: Reduction of 7-amino-3-methylphtalide 6 to 2-amino-6-ethylbenzoic acid 3

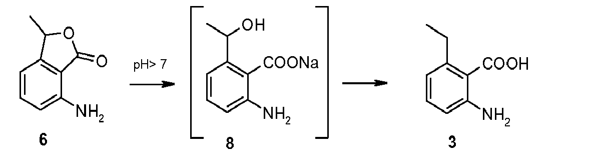

It was found that the lactone **6** is very resistant to reduction into 2-amino-6-ethylbenzoic acid **3** under acidic or neutral conditions but is easily reduced to 2-amino-6-ethylbenzoic acid **3** in water at a pH of 7 or above, probably following a path via the ring-opened 2-amino-6-(1-hydroxy-ethyl)-benzoic acid sodium salt **8.** 2-amino-6-(1-hydroxy-ethyl)-benzoic acid sodium salt **8** has earlier been described in US 2003/0114690 A1.

The hydrogenation catalyst used for this reduction of 7-amino-3-methylphtalide **6** into 2-amino-6-ethylbenzoic acid **3** should be either Raney nickel (RaNi) or a palladium based catalyst, such as palladium on carbon (Pd/C). According to some embodiments, Raney nickel is preferred. It has been found that these catalysts are effective catalysts at a pH of 7 or above for reducing 7-amino-3-methylphtalide **6** into 2-amino-6-ethylbenzoic acid **3.** It has also been found that platinum based catalysts cannot be used for this reduction (Table 1).

According to some embodiments it is preferred that the reduction of 7-amino-3-methylphtalide **6** into 2-amino-6-ethylbenzoic acid **3** is done in water and with pH-adjustment to a pH of 7 or above. However, it is also possible to use a mixture of water and one or more water miscible solvent(s), again at a pH of 7 or above. If a mixture is used, it is preferred according to some embodiments that it consists of at least 80% water. One of the reasons water or a mixture with a high content of water may be preferred is based on safety, since from a safety point of view use of non-flammable solvents such as water for hydrogenations using RaNi or palladium is a clear advantage.

7-amino-3-hydroxy-3-methylphtalide **7** was reduced with hydrogen gas using Raneynickel in buffered water at pH 6, pH 7, and pH 8 respectively (Table 1). The hydrogen pressure was 3.9 bar and the temperature 90°C. The reaction was stopped after 1 hour and evaluated by TLC. It was evident that the reduction yielded mainly 7-amino-3-methylphtalide **6** when pH was 6. At pH 7 a mixture of **6** and **3** was formed, and at pH 8 the desired 2-amino-6-ethylbenzoic acid **3** was formed in almost quantitative yield. Therefore, the pH should be 7 or above, more preferably the pH should be 8 or above and most preferably the pH is 10-12.

The pH adjustment can be done by any conventional method known to the skilled person that does not adversely affect the reduction of 7-amino-3-methylphtalide **6** into 2-amino-6-ethylbenzoic acid **3.** Preferably the pH is adjusted to the desired value by addition of an aqueous base. The aqueous base may for example be an aqueous solution of an alkali metal hydroxide, alkaline earth metal hydroxide, alkali metal carbonate or alkaline earth metal carbonate. According to some embodiments, aqueous sodium hydroxide or potassium hydroxide is preferred.

The temperature at which the reduction is performed is not critical. Preferably it is 20-100 °C, more preferably it is 50-100 °C, even more preferably it is 80-100 °C.

When the hydrogen source is hydrogen gas, the pressure is not critical. For example it may be 1-100 bar. The safety aspect should also be considered when choosing the hydrogen pressure. By increasing the pressure, it may be possible to reduce the temperature and still obtain a reasonable reaction rate. A suitable pressure may therefore be 2-5 bar.

2-amino-6-ethylbenzoic acid **3** may be isolated with any suitable standard method, such as extraction, precipitation or evaporation. One example of how this may be done is outlined in Example 1.

The hydrogenation is preferably continued until the mixture consists mainly or of 2-amino-6-ethylbenzoic acid.

In comparison with the other known methods this new route to 2-amino-6-ethylbenzoic acid **3** is superior and will be utilized for providing starting material for future manufacturing campaigns of paquinimod.

As mentioned above it is highly likely that the reduction of 7-amino-3-methylphtalide **6** into 2-amino-6-ethylbenzoic acid **3** takes the route via the intermediate **8**, 2-amino-6-(1-hydroxy-ethyl)-benzoic acid sodium salt (or potassium salt if KOH was used for pH-adjustment. Compound **6** has a low intrinsic solubility in water but upon heating and addition of a base, e.g. NaOH, compound **6** dissolves and TLC indicates formation of compound **8**. Compound **8** can be prepared pure by treatment of **6** with ethanolic KOH as described in the examples. The 7-amino-3-methylphtalide **6** that is reduced to 2-amino-6-ethylbenzoic acid **3** as described above may be formed from 2-acetyl-6-nitrobenzoic acid **5** by reduction in a water miscible solvent, or in a mixture of such a solvent and water, or in water alone, using a hydrogen source selected from the group consisting of hydrogen gas, formic acid derivatives and cyclohexene, and a hydrogenation catalyst selected from Raney nickel, palladium based catalysts, and platinum based catalysts: However, Raney nickel may not be used as the hydrogenation catalyst if the reduction takes place in only water. Furthermore, when the reduction takes place in water or in a water containing mixture the pH shall be adjusted to 7 or higher. The reduction of 2-acetyl-6-nitrobenzoic acid **5** to 7-amino-3-methylphtalide **6** takes place via intermediate formation of 7-amino-3-hydroxy-3-methylphtalide **7**.

According to some embodiments it is preferred that the reduction of 2-acetyl-6-nitrobenzoic acid **5** into 7-amino-3-hydroxy-3-methylphtalide **7** and 7-amino-3-methylphtalide **6** is done in water. However, it is also possible to use a mixture of water and one or more water miscible solvent(s). If a mixture is used, it is preferred according to some embodiments that it consists of at least 80% water. One of the reasons water or a mixture with a high content of water may be preferred is based on safety, since from a safety point of view use of non-flammable solvents such as water for hydrogenations using RaNi, platinum or palladium is a clear advantage. It is also possible to use only a water miscible solvent, and in this case there is no need to adjust the pH. Examples of suitable water miscible solvents are ethanol, n-propanol and isopropanol. Other examples are methanol and dioxane. The use of RaNi in ethanol or Pd/C in dioxane has also earlier been described (Luthy et al) for the reduction of **5** into **6**, presumably via intermediate **7**.

The choice of catalyst depends i.a. on the conditions used for the reaction. For example, it has been found that when this reduction is done in water, Raney nickel is the preferred catalyst at a pH of 7 or higher for the reduction of 7-amino-3-hydroxy-3-methylphtalide **7** to 7-amino-3-methylphtalide **6** (and further to 2-amino-6-ethylbenzoic acid **3**), but Raney nickel is not a good choice at a pH of 7 or above for reduction of 2-acetyl-6-nitrobenzoic acid **5** to 7-amino-3-hydroxy-3-methylphtalide **7** since inhibiting byproducts then will form.

When the reduction of **5** into a mixture of **7** and **6** is performed in water it is important to have a pH of 7 or above in order to ensure solubility of **6**. If pH is less than 7, precipitation of **6** during the reduction may complicate the process.

The temperature at which the reduction is performed is not critical. Preferably it is 20-100 °C, more preferably it is 20-80 °C, and even more preferably it is approximately 70°C.

When the hydrogen source used for the reduction of 2-acetyl-6-nitrobenzoic acid into 7-amino-3-methylphtalide **6** and 7-amino-3-hydroxy-3-methylphtalide **7** is hydrogen gas, the pressure is not critical. For example it may be 1-100 bar. The safety aspect should also be considered when choosing the hydrogen pressure. By increasing the pressure, it may be possible to reduce the temperature and still obtain a reasonable reaction rate. A suitable pressure may therefore be 2-5 bar.

The reduction of 2-acetyl-6-nitrobenzoic acid is preferably continued until at least 85 %, and more preferably at least 95 % of the 2-acetyl-6-nitrobenzoic acid **5** has been reduced into a mixture consisting mainly of 7-amino-3-hydroxy-3-methylphtalide **7** and 7-amino-3-methylphtalide **6**.

Other ways of forming 7-amino-3-methylphtalide 7 from 2-acetyl-6-nitrobenzoic acid **6** have been described by Luthy et al.

Preferably, the complete reduction of 2-acetyl-6-nitrobenzoic acid **5** into 2-amino-6-ethylbenzoic acid **3** is done in the same reaction vessel, following Scheme 3 illustrated below, instead of performing separate reductions with isolation of intermediates.

### Scheme 3: Reduction of 2-acetyl-6-nitrobenzoic acid 5 to 2-amino-6-ethylbenzoic acid 3

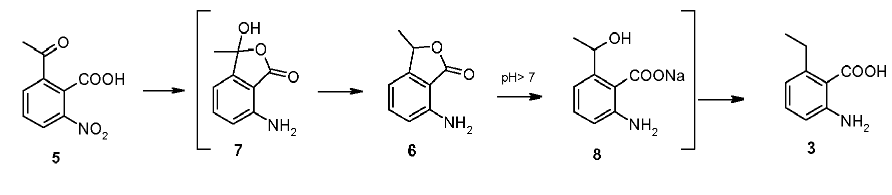

Alternatively, the 7-amino-3-methylphtalide **6** that is reduced to 2-amino-6-ethylbenzoic acid **3** as described above may be formed from 3-methyl-7-nitrophtalide **9** by conventional methods for reduction of aromatic nitro groups into amine groups. Luthy et al describes reduction of **5** into **9** using sodium borohydride. After conventional reduction of **9** into **6**, e.g. by using catalytic hydrogenation at room temperature with palladium catalyst, further reduction into **3** can be performed as described above or preferable the complete reduction of **9** to **3** (presumably via intermediates **6** and **8**) is performed in water or in mixtures of water and a water-miscible solvent, with the addition of a base to ensure a pH above 7 for facilitating formation of the intermediate **8**, using a hydrogen source selected from the group consisting of hydrogen gas, formic acid derivatives and cyclohexene, and a hydrogenation catalyst selected from Raney nickel or palladium based catalysts. A platinum based hydrogenation catalyst cannot be used.

Another way of forming 7-amino-3-methylphtalide **6** from 3-methyl-7-nitrophtalide **9** is described in US 2003/0114690 A1.

Preferably, the whole reduction from 3-methyl-7-nitrophtalide **9** into 2-amino-6-ethylbenzoic acid **3** is done at the same time, following Scheme 4 illustrated below, instead of performing separate reductions.

### Scheme 4: Reduction of 3-methyl-7-nitrophtalide 9 to 2-amino-6-ethylbenzoic acid 3

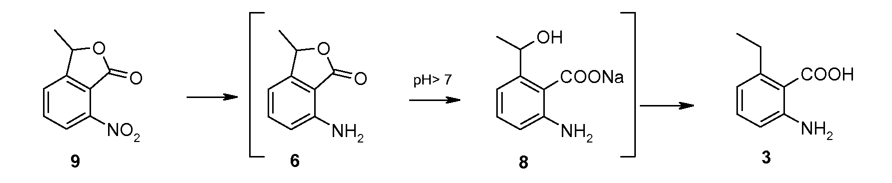

The invention will be further described in the examples that follow.

Of the compounds used in the Examples some have been described before, these are 2-amino-6-ethylbenzoic acid **3** (US 4,873,232), 2-acetyl-6-nitrobenzoic acid **5**, 7-amino-3-methylphtalide **6**, 7-amino-3-hydroxy-3-methylphtalide **7**, 2-amino-6-(1-hydroxy-ethyl)-benzoic acid sodium salt (US 2003/0114690 A1) and 3-methyl-7-nitrophthalide **9**. Compounds **5**, **6**, **7** and **9** have been described by Luthy et **al**.

### EXAMPLE 1

Reactions were performed at 80 °C for 1 h and at a pressure of 3.9 bar. 1 mmol substrate was hydrogenated under the conditions described in Table 1 below. The amounts of catalysts were PtO₂ (30 mg), 10 % Pd/C (30 mg), Raney 2800 nickel (50 mg wet susp.). Solvent volume was 3.0 mL. The catalysts were filtered off and the mixtures were diluted with water to a final volume of 5.0 mL. Analysis by UPLC gave the molar yield for each of the compounds **5**, **7**, **6**, and **3**.

**Table 1. Small-scale Hydrogenation Experiments**

| Substrate (1 mmol) | Conditions | Catalyst | **5** mol % | **7** mol % | **6** mol % | **3** mol % | Detected sum of **5**, **7**, **6**, and **3** mol %^{a} |
|---|---|---|---|---|---|---|---|
| **5** | water pH=12 | Pt | 0 | 87 | 7 | 0 | 94 |
| **5** | water pH=12 | Pd | 0 | 49 | 10 | 7 | 66 |
| **5** | water pH=12 | RaNi | 48 | 23 | 0 | 0 | 71 |
| **7** | water pH=12 | Pt | 0 | 73 | 4 | 0 | 77 |
| **7** | water pH=12 | Pd | 0 | 39 | 11 | 12 | 62 |
| **7** | water pH=12 | RaNi | 0 | 0 | 25 | 72 | 97 |
| **6** | water pH=12 | Pt | 0 | 0 | 94 | 2 | 96 |
| **6** | water pH=12 | Pd | 0 | 0 | 0 | 100 | 100 |
| **6** | water pH=12 | RaNi | 0 | 0 | 21 | 76 | 97 |
| **5** | water pH=1 | Pt | 0 | 9 | 0 | 0 | 9 |
| **5** | water pH=1 | Pd | 0 | 18 | 47 | 2 | 67 |
| **5** | water pH=1 | RaNi ^{b} | 50 | 23 | 0 | 0 | 73 |
| **5** | n-propanol | RaNi | 0 | 32 | 26 | 3 | 61 |
| **5** | water neutral | RaNi | 12 | 34 | 12 | 0 | 58 |
| **5** | n-propanol | Pd | 0 | 0 | 55 | 9 | 64 |
| **9** | water pH=12 | Pd | 0 | 0 | 0 | 95 | 95 |
| **7** | EtOH/wat er | RaNi | 0 | 49 | 42 | 4 | 95 |
| **7** | EtOH/wat er pH=12 | RaNi | 0 | 0 | 61 | 38 | 99 |
| **7** | Water buffered at pH 6 | RaNi | 0 | 0 | 95 | 5 | Note c |
| **7** | Water buffered atpH7 | RaNi | 0 | 0 | 50 | 50 | Note c |
| **7** | Water buffered at pH 8 | RaNi | 0 | 0 | 5 | 95 | Note c |

| | | | | | | | |
|---|---|---|---|---|---|---|---|
| Notes: a) The yield of lactone **6** was probably underestimated in some cases due to an equilibrium with the ring opened lactone **8** (see Scheme 3) at a pH of 7 or above. This is reflected in a low sum of **5**, **7**, **6**, and **3**. In one case (reduction of **5** with Pt as catalyst and pH=1) it was evident that saturation of the benzene ring occurred and that the products had low UV-absorption in UPLC. b) The catalyst was completely dissolved. c) Conditions as described but the temperature used was90 °C .The yields were estimated by comparison with reference compound on TLC (silica, eluted with chloroform/methanol 10/1) using UV-detection. | | | | | | | |

### EXAMPLE 2: 2-amino-6-ethylbenzoic acid 3

From **5** using Pt first, then RaNi; 2-acetyl-6-nitrobenzoic acid **5** (Luthy et al) (5.00 g, 23.9 mmol) was dissolved in 1 M NaOH(aq) (25 mmol, 25 mL) and water (25 mL). The pH was adjusted to approx. 12 and platinum(IV)oxide (100 mg) was added. The mixture was hydrogenated at 70 °C, P= 3.9 bar, for 3 h (complete conversion into **7** according to TLC) and RaNi (Raney 2800 nickel, 1.5 g wet suspension) was added. The hydrogenation was continued at 90 °C, P=3.9 bar, for 3 h and then stopped. The catalysts were filtered off and pH was adjusted to approx. 3.0-3.5 with hydrochloric acid. The mixture was extracted with EtOAc (2x50 mL) and the combined extracts were dried with sodium sulfate and filtered (at large-scale drying the extract by means of azeotropic removal of water, or washings with brine, are perhaps better). The volume of the extract was reduced to approx. 20 mL by evaporation. Precipitation of **3** was induced by addition of n-heptane (100 mL). The crystalline suspension (very thick!) was stirred for 15 h in order to become more easily filtered. Alternatively, ultrasonication of the thick mixture for some minutes breaks the tiny needle-shaped crystals and the mixture becomes more easily filtered. The mixture was cooled on an ice-bath and the crystals were collected by filtration, washed with n-heptane, and dried in vacuum to give pure **3** as a white crystalline material that darkens slightly upon storage (3.55 g, 90 % yield): mp 107-109 °C; ¹H NMR (CDCl₃) δ 7.50 (bs, 2H), 7.19 (t, 1H, *J* = 7.8 Hz), 6.62 (d, 1H, *J* = 7.4 Hz), 6.58 (d, 1H, *J* = 8.1 Hz), 2.96 (q, 2H, *J* = 7.5 Hz), 1.27 (t, 3H, *J* = 7.5 Hz).

### EXAMPLE 3

From **5** using Pd/C first, then RaNi; By following the procedure above, but substituting platinum(IV)oxide with 10% Pd/C (300 mg), compound **3** was isolated as a pure and off-white crystalline material (3.41 g, 86 % yield).

### EXAMPLE 4

From **9** using Pd/C; 3-Methyl-7-nitrophtalide **9** (5.00 g, 25.8 mmol), 2 M NaOH(aq) (27 ml, 54 mmol), water (23 mL), and 10 % Pd/C (500 mg), were hydrogenated at 90 °C, P=3.9 bar, for 8 h. Compound **3** was isolated, using the above procedure, as a pure and white crystalline material (3.62 g, 85 % yield).

### EXAMPLE 5: 2-amino-6-(1-hydroxy-ethyl)-benzoic acid 8 (as the potassium salt)

7-amino-3-methylphthalide **6** (530 mg) was dissolved in ethanol (99.5 %, 2 mL) and a saturated solution of KOH(s) in ethanol (99.5 %, 1 mL) was added and the mixture was heated at 60 °C for 2 min. After cooling to room temperature acetone (7 mL) was added slowly and the precipitate was filtered off, washed with acetone, and dried, to give 2-amino-6-(1-hydroxy-ethyl)-benzoic acid potassium salt **8** as a hygroscopic white powder. Yield 493 mg. H-NMR in d-DMSO; 8.13 (1H, d, OH), 6.79 (1H, t, aromatic H), 6.46 (1H, d, aromatic H), 6.35 (1H, d, aromatic H), 5.63 (2H, bs, NH2), 4.51 (1H, pentet), 1.31 (3H, d, CH3).

### References

Hansch, C. J. Org. Chem. 1955, 20, 1026.
Jansson, K.; Fristedt, T.; Olsson, A.; Svensson, B.; Jönsson, S. J. Org. Chem. 2006, 71, 1658*.*
Jönsson, S.; Andersson, G.; Fex, T.; Fristedt, T.; Hedlund, G.; Jansson, K.; Abramo, L.; Fritzson, I.; Pekarski, 0.; Runström, A.; Sandin, H.; Thuvesson, I.; Björk, A. J. Med. Chem. 2004, 47, 2075
Luthy, C.; Zondler, H.; Rapold, T.; Seifert, G.; Urwyler, B.; Heinis, T.; Steinrucken, H.; Allen, J. Pest Manag. Sci. 2001, 57, 205.
Newman, M.; Kannan, R. J. Org. Chem. 1976, 41, 3356.

## Claims

1. A method for the production of 2-amino-6-ethylbenzoic acid wherein 7-amino-3-methylphtalide or 7-amino-3-hydroxy-3-methylphtalide is reduced to 2-amino-6-ethylbenzoic acid in water or in a mixture of a water miscible solvent and water at a pH adjusted to 7 or higher using a hydrogenation catalyst selected from Raney nickel and palladium based catalysts and using a hydrogen source selected from the group consisting of hydrogen gas, formic acid derivatives and cyclohexen.

2. A method according to claim 1, wherein only water is used.

3. A method according to claim 1 or 2, wherein the pH is adjusted to 10-12.

4. A method according to any one of claims 1-3, wherein the hydrogenation catalyst is Raney nickel.

5. A method according to any one of claims 1-4, wherein the hydrogen source is hydrogen gas.

6. A method according to any one of the claims 1-5 wherein the 7-amino-3-methylphtalide or 7-amino-3-hydroxy-3-methylphtalide that is reduced to 2-amino-6-ethylbenzoic acid is formed from 2-acetyl-6-nitrobenzoic acid by reduction of 2-acetyl-6-nitrobenzoic acid in a water miscible solvent, or in a mixture of such a solvent and water, or in water alone, using a hydrogen source selected from the group consisting of hydrogen gas, formic acid derivatives and cyclohexen, and a hydrogenation catalyst selected from Raney nickel, palladium based catalysts, and platinum based catalysts, with the proviso that the hydrogenation catalyst may not be Raney nickel when water alone is used, and further with the proviso that when water or a water containing mixture is used the pH is adjusted to 7 or higher.

7. A method according to claim 6 wherein the pH used for reduction of 2-acetyl-6-nitrobenzoic acid is adjusted to 10-12 when water or a water containing mixture is used.

8. A method according to claim 6 or 7 wherein a water miscible solvent is used and wherein the water miscible solvent is ethanol, n-propanol or isopropanol.

9. A method according to claim 6 or 7 wherein water alone is used.

10. A method according to any one of claims 6-9, wherein the hydrogenation catalyst used for reduction of 2-acetyl-6-nitrobenzoic acid to 7-amino-3-methylphtalide or 7-amino-3-hydroxy-3-methylphthalide is palladium or platinum.

11. A method according to any one of the claims 1-5, wherein the 7-amino-3-methylphtalide that is reduced to 2-amino-6-ethylbenzoic acid is formed from 3-methyl-7-nitrophtalide by reduction of the nitro substituent.

12. A method according to claim 11, wherein a palladium or nickel based catalyst is used as hydrogenation catalyst and wherein the reduction is performed at a pH of 7 or higher.

13. 2-Amino-6-ethylbenzoic acid produced with the method according to any one of claims 1- 12.

14. Use of 2-amino-6-ethylbenzoic acid according to claim 13 or produced according to any one of the claims 1-12 in the production of N,5-diethyl-1,2-dihydro-4-hydroxy-1-methyl-2-oxo-N-phenyl-3-quinolinecarboxamide (paquinimod).
